# EUROPÄISCHE PATENTSCHRIFT

(11) **EP 1 658 012 B1**
(45) Veröffentlichungstag und Bekanntmachung des Hinweises auf die Patenterteilung: **14.03.2007**
(21) Anmeldenummer: 04763754.1
(22) Anmeldetag: 03.08.2004
(51) Int. Cl.: A61B 17/50

(54) **VORRICHTUNG ZUR ENTFERNUNG VON ZECKEN**
DEVICE FOR THE REMOVAL OF TICKS
DISPOSITIF SERVANT A RETIRER DES TIQUES

(30) Priorität: 09.08.2003 DE 10337023; 30.06.2004 DE 102004031682
(43) Veröffentlichungstag der Anmeldung: 24.05.2006
(73) Patentinhaber: Meinhold, Matthias, 90425 Nürnberg (DE); Kuffner, Hermann, 91126 Schwabach (DE)
(72) Erfinder: Meinhold, Matthias, 90425 Nürnberg (DE); Kuffner, Hermann, 91126 Schwabach (DE)
(74) Vertreter: Meyer, Enno
(86) Internationale Anmeldenummer: PCT/EP2004/008696
(87) Internationale Veröffentlichungsnummer: WO 2005/013837

(56) Entgegenhaltungen:
- CH-A- 255 260
- DE-A- 10 023 740
- DE-A- 10 148 742
- DE-A- 19 860 172
- DE-U- 20 009 056
- DE-U- 29 719 008

## Beschreibung

Die Erfindung betrifft eine Vorrichtung zum Entfernen von Parasiten oder Zecken aus der Haut eines Wirts nach dem Oberbegriff des Anspruchs 1.

Erkrankungen insbesondere des Menschen infolge eines Zeckenbisses breiten sich erschreckend aus. Die rechtzeitige und sachgemäße Entfernung der Zecke aus der Haut des Wirts ist Prävention und Therapie zugleich, wobei unter sachgemäßer Entfernung das Herausziehen der unbeschädigten Zecke aus dem Wirt verstanden wird, ohne dass die Zecke gequetscht oder anderweitig irritiert und eine Infektion dadurch erst verursacht wird.

Herkömmliche Verfahren zum Entfernern von Zecken aus der menschlichen Haut bewirken eine Quetschung der Zecke, wenn diese fest erfasst und entfernt werden soll. Insbesondere bei Parasiten mit infektiösem Inhalt bewirkt diese Art der Entfernung das Herausquetschen infektiösen Materials und löst dadurch erst die Erkrankung aus. Oberstes Ziel neben der Expositionsprophylaxe ist aber das schonende Entfernen des unversehrten Parasiten.

Herkömmlicherweise werden Zecken mit einer Zange oder Pinzette entfernt. Dabei lässt sich das Quetschen der Zecke oder ihrer Teile nicht vermeiden. Durch den Einsatz eines Bindfadens kann dieses Risiko bei sachgemäßer Anwendung gemindert werden, doch ist dieses Verfahren mühselig, zeitraubend und nicht immer von Erfolg gekrönt. Insbesondere an schwierigen Körperstellen wie der Achselhöhle stößt die Fadenmethode an ihre Grenzen.

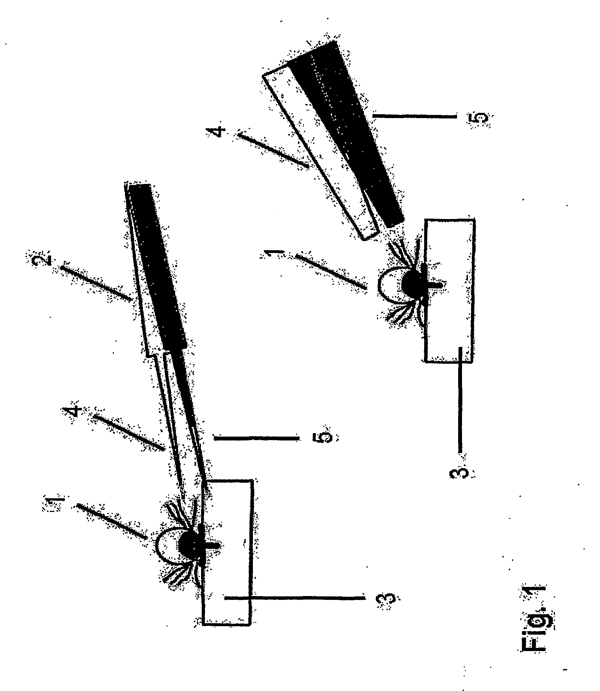

Bei der Entfernung der Zecke mittels einer Pinzette werden in den illustrierenden Skizzen der Übersicht halber häufig das Größenverhältnis von Pinzette, Fingernagel oder Zange zur Zecke vernachlässigt. So entsteht der Eindruck, die Zecke werde am Kopf gepackt und daran herausgezogen. Tatsächlich aber misst die Zangenspitze meist ein Vielfaches der Zecke selbst, so dass es unvermeidlich zur Quetschung des Zeckenkörpers kommt, was das Entleeren potentiell infektiösen Magen- Darminhaltes der Zecke zur Folge hat. Dies ist in Fig. 1 dargestellt, wobei der obere Teil der Fig. 1 das häufig dargestellte, aber falsche Größenverhältnis von Pinzette und Zecke zeigt, während der untere Teil der Fig. 1 das richtige Größenverhältnis darstellt, aus dem ersichtlich ist, dass im wesentlichen eine Quetschung der Zecke aufgrund der großen Pinzettenarme erfolgt.

Folgt man den Empfehlungen der medizinischen Fachliteratur bzw. des Internets, so wird zum korrekten Entfernen einer Zecke eine spitze Pinzette, eine Zeckenzange, ein Bindfaden oder der Fingernagel empfohlen. Während das Entfernen mit dem Bindfaden zwar zecken- und damit patientenschonend, aber zeitaufwendig und nicht an allen Körperpartien praktikabel ist, bedingen starre oder wenig flexible Zangen inklusive des Fingernagels unweigerlich eine Quetschung des Zeckenkörpers und damit ein erhöhtes Infektionsrisiko, wie dies oben diskutiert wurde.

Ferner beschreibt Druckschrift DE 19860172 A1, gemäß dem oberbegriff des ersten Anspruchs, eine automatische Zeckenzange. Diese weist eine Greifereinrichtung auf, die durch eine Greiferhülse willkürlich zu öffnen und zu schließen ist, und die dem Ergreifen einer Zecke dient. Die Zeckenzange ist außerdem mit einem Drehantrieb versehen, mit dem die Greifereinrichtung drehend antreibbar ist. Zusätzlich ist eine durch eine Druckfeder gebildete Linearantriebseinrichtung vorgesehen, mit der die Greifereinrichtung linear von der Haut des Wirtsorganismus weg bewegbar ist. Die Koordinierung von Drehbewegung und linearer Auszugsbewegung der Greifereinrichtung wird von einer Steuereinrichtung übernommen, zu der beispielsweise Auslösefinger und Stützfinger oder eine Rastzunge und ein Auslöseschieber gehören, der durch Drehbewegung der Greifereinrichtung zum Lösen der Rastzunge aktiviert wird. Das Ausziehen einer Zecke erfolgt somit in wenigen Sekunden automatisch. Zwar sind bei der bekannten Vorrichtung Greif- und Drehmechanismus miteinander verknüpft, jedoch sind die Greifer starr, so dass Infektionsmaterial in den Wirtsorganismus gequetscht werden kann.

Die DE 19827651C1 zeigt eine Vorrichtung zum Entfernen von Zecken, welche Greifarme aufweist, die an einer Stelle zwischen zwei aus einem Gehäuse herausragenden Griffstücken und Greifbacken über einen Stützsteg einstückig miteinander verbunden sind. Die inneren Enden der Greifarme stützen sich auf Schrägflächen im Gehäuse ab. Durch die Eigenelastizität der aus elastischem Kunststoff einstückig gespritzten Greifarme werden die Greifbacken bei unbetätigter Vorrichtung mit vorgegebener Schließkraft zusammengedrückt. Die bekannte Vorrichtung zum Entfernen von Zecken arbeitet zwar mit einer Kunststoffpinzette, jedoch ist auch hier die Gefahr gegeben, dass die zwei spitzen Greifer die Zecke quetschen.

Der Erfindung liegt daher die Aufgabe zugrunde, eine Vorrichtung der gattungsgemäßen Art zu schaffen, die eine sachgemäße Entfernung einer Zecke oder eines derartigen Parasiten vom Wirt ermöglicht.

Diese Aufgabe wird durch eine Vorrichtung zum Entfernen von Parasiten oder Zecken mit den Merkmalen des Anspruchs 1 gelöst. Bevorzugte Ausgestaltungen der Erfindung sind Gegenstand der Unteransprüche.

Die erfindungsgemäße Vorrichtung, die insbesondere zum Entfernen von Parasiten oder Zecken aus der Haut von Tieren und Menschen dient und auch als Zeckenfänger bezeichnet wird, umfasst ein Gehäuse, ein spreizbares Greifwerkzeug, eine Spreizvorrichtung zum Spreizen des Greifwerkzeugs und eine Drehvorrichtung zur Drehung des Greifwerkzeugs um die Achse der Vorrichtung, wobei das Greifwerkzeug in seinem ungespreizten Zustand einen im wesentlichen geschlossenen Hohlraum zur Aufnahme des Parasiten oder der Zecke umschließt. Mit anderen Worten, der im Innern des Greifwerkzeugs ausgebildete Hohlraum wird im wesentlichen vollständig von dem Greifwerkzeug im geschlossenen Zustand umfasst.

Die Vorrichtung weist weiterhin eine in axialer Richtung der Vorrichtung wirkende Druckvorrichtung zur Betätigung der Spreizvorrichtung und der Drehvorrichtung des Greifwerkzeugs auf. Dabei bewirkt eine erste Betätigung der Druckvorrichtung eine Spreizung der Greifwerkzeugs durch das Spreizvorrichtung und eine zweite Betätigung der Druckvorrichtung bewirkt eine Drehung der Greifwerkzeugs. Weiterhin wird eine Schließung des Greifwerkzeugs durch die erste oder die zweite Betätigung der Druckvorrichtung bewirkt.

In einer ersten bevorzugten Ausführungsform erfolgt die erste Betätigung der Druckvorrichtung in axialer Richtung in die Vorrichtung hinein und die sich an die erste Betätigung anschließende zweite Betätigung in axialer Richtung erfolgt aus der Vorrichtung heraus. Mit anderen Worten, in der bevorzugten Ausführungsform, die in Form einem Druckbleistift ähnelt, wird die Druckvorrichtung über einen Druckstift betätigt und bei Eindrücken des Druckstiftes erfolgt die Spreizung des Greifwerkzeugs. Beim Loslassen des Druckstiftes, d.h. Betätigung in die entgegengesetzte Richtung zur ersten Betätigung, erfolgt die Drehung des Greifwerkzeuges . Das Schließen des Greifwerkzeuges kann entweder am Ende der ersten Betätigung oder zu Beginn der zweiten Betätigung des Druckstiftes erfolgen.

In einer zweiten bevorzugten Ausführungsform erfolgt die erste Betätigung der Druckvorrichtung in axialer Richtung ebenfalls in die Vorrichtung hinein und die sich an die erste Betätigung anschließende zweite Betätigung erfolgt ebenfalls in axialer Richtung in die Vorrichtung hinein. Mit anderen Worten, im Fall einer Ausbildung der Vorrichtung ähnlich zu einem Druckbleistift erfolgt die Spreizung durch ein erstes Eindrücken des Druckstiftes. Ein weiteres Eindrücken des Druckstiftes bewirkt eine Drehung des Greifwerkzeugs. Das Schließen des Greifwerkzeugs kann auch hier zu Ende der ersten Betätigung oder zu Beginn der zweiten Betätigung erfolgen.

Vorzugsweise ist das Greifwerkzeug aus zwei oder mehreren Segmenten gebildet, wobei insbesondere zwei, drei oder vier Segmente bevorzugt sind. Die Segmente des Greifwerkzeugs sind vorzugsweise elastisch ausgebildet.

Der Hohlraum des Greifwerkzeugs kann gebildet werden, indem die Segmente des Greifwerkzeugs zur Spitze des Greifwerkzeugs hin sich verjüngende und nach innen gerichtete Greifbacken bilden. Ferner weisen die Segmente des Greifwerkzeugs vorzugsweise eine Federkraft auf, gegen welche die Spreizung der Segmente erfolgt. Mit anderen Worten, die Segmente sind so ausgebildet, dass sie aufgrund der eigenen Federkraft im geschlossenen Zustand den inneren Hohlraum bilden und ihn umschließen, so dass eine Spreizung der Segmente, d.h. ein Öffnen des Greifwerkzeugs, gegen die Federkraft der Segmente erfolgt.

Ferner kann das Greifwerkzeug mit einem Klebstoff beschichtet sein und/oder Widerhaken aufweisen. Weiterhin kann die Vorrichtung eine mit dem Greifwerkzeug verbundene Saugeinrichtung aufweisen. Diese zusätzlich möglichen Maßnahmen dienen zur verbesserten Fixierung des Zecke.

Das Greifwerkzeug ist vorzugsweise aus einem elastischen bzw. flexiblen Material wie Silikon, insbesondere medizinischem Silikon, oder aus einem geeignetes elastischen kunststoffbeschichteten Material gebildet.

Weiterhin weist vorzugsweise die Vorrichtung eine Einrichtung zum Lähmen oder Abtöten der Parasiten auf. Dies kann beispielsweise durch ein Abtöten mittels Strom oder LaserStrahl erfolgen sowie durch Lähmung oder Abtötung über ein geeignetes Pharmakon.

Vorzugsweise ist das Greifwerkzeug austauschbar, so dass aus Hygienegründen das Greifwerkzeug nach einmaligem Gebrauch entsorgt wird, um eine Ansteckungsgefahr durch ein bereits gebrauchtes Greifwerkzeug zu vermeiden. Insbesondere ist das Greifwerkzeug eine von der übrigen Mechanik abgetrennte Einheit, die beispielsweise mittels Bajonettverschluß oder ähnlichem mit der Drehvorrichtung verbunden ist. Dadurch besteht eine hygienische Abgrenzung des Einmalgreifwerkzeugs zur wiederverwendbaren Mechanik des Zeckenfängers. Ferner steht das Greifwerkzeug in verschiedenen Größen und Materialstärken zum Austausch zur Verfügung, so dass ein für die Größe des Parasiten passendes Greifwerkzeug zum Einsatz gelangen kann. Dadurch wird die Gefahr einer Quetschung des Parasiten weiter verringert.

Vorzugsweise weist die Vorrichtung eine Abwurfeinrichtung zum Abwerfen des Greifwerkzeugs auf. Damit ist es möglich, dass das Greifwerkzeug von der Vorrichtung abgetrennt wird, nachdem die Zecke sich im Hohlraum des Greifwerkzeuges befindet, oder wenn ein Greifwerkzeug einer anderen Größe verwendet werden muss. Insbesondere kann die Abwurfeinrichtung das Greifwerkzeug mit Beendigung des Vorgangs der Entfernung eines Parasiten von der Vorrichtung lösen.

Dem beschriebenen Rotationsprinzip liegt die Beobachtung zugrunde, dass die Drehung der Zecke selbst oder des Greifwerkzeuges auf dem Zeckenkörper die Zecke veranlasst, sich vom Wirt zu lösen.

Ferner ist der Zeckenfänger so konzipiert, dass eine Einhandbedienung möglich und damit auch an "schwierigen" Körperpartien wie z. B. der Achselhöhle zu verwenden ist.

Vorteilhafterweise stehen die Greifwerkzeuge in mehreren Größen zur Verfügung, um von der kleinsten Nymphe bis zum vollgesogenem, erwachsenen Zeck die verschiedensten Zeckengrößen aufzunehmen. Verschiedene Materialstärken sollen den unterschiedlichen Verhältnissen bei Mensch und Tier gerecht werden. Ferner kann das Greifwerkzeug einen Feuchtigkeitsspender aufweisen, so dass die vom Greifwerkzeug üblicherweise lebend gefangene Zecke im lösbaren Einmalgreifwerkzeug einige Zeit überleben kann, beispielsweise um zu einer Untersuchung geschickt zu werden.

Bevorzugte Ausführungsformen der Erfindung werden nachfolgend anhand der Zeichnungen dargestellt. Dabei zeigen
- Fig. 1: ein fiktives und ein reales Größenverhältnis zwischen einer Zecke und einer Pinzette,
- Fig. 2: ein erstes Ausführungsbeispiel der erfindungsgemäßen Vorrichtung,
- Fig. 3: ein zweites Ausführungsbeispiel der erfindungsgemäßen Vorrichtung,
- Fig. 4: verschiedene Segmentvarianten des Greifwerkzeugs, und
- Fig. 5: die Anwendung der ersten Ausführungsform der Erfindung.

Fig. 1 wurde bereits im einleitenden Teil der Beschreibung erläutert und zeigt in seinem oberen Teil ein Abbildung des falschen Größenverhältnisses zwischen einer Zecke 1, die sich in die Haut 3 eines Wirts gebohrt hat, und einer Pinzette 2, wie sie in der Literatur zu finden ist. Der untere Teil der Fig. 1 spiegelt demgegenüber das reale Größenverhältnis wieder und zeigt deutlich, dass die Pinzettenarme 4, 5 im Vergleich zur Zecke 1 überdimensioniert sind, wodurch eine Quetschung der Zecke 1 beim Entfernen unvermeidlich ist.

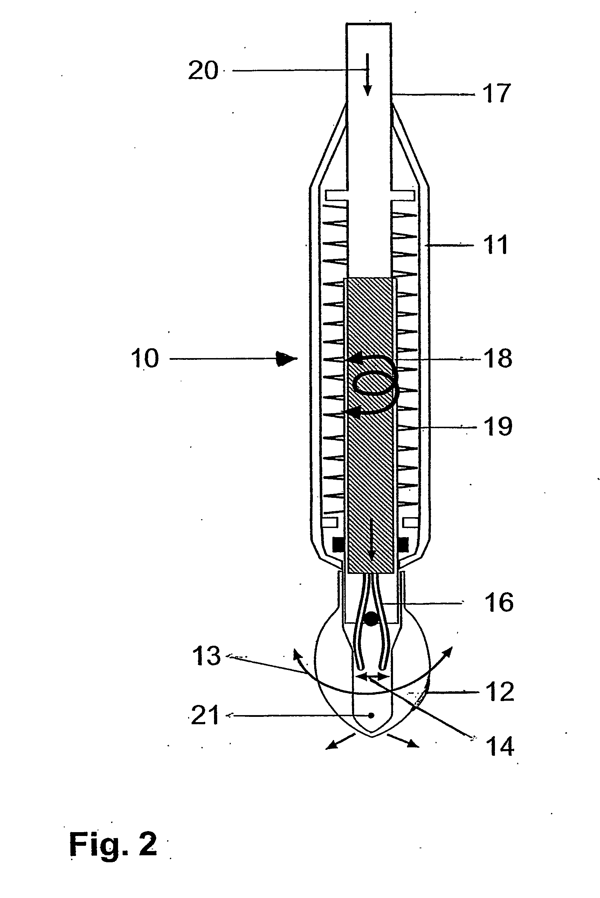

Fig. 2 zeigt eine erste Ausführungsform des erfindungsgemäßen Zeckenfängers 10, der ein stiftförmiges Gehäuse 11 aufweist, an dessen unteren Ende ein spreizbares und drehbares Greifwerkzeug 12 angeordnet ist, wie dies durch die Pfeile 13, 14 und 15 angedeutet ist. Dabei symbolisiert Pfeil 13 die Drehrichtung des Greifwerkzeugs um die Längsachse (nicht dargestellt) der Vorrichtung 10, Pfeil 14 die Spreizrichtung zum Spreizen des Greifwerkzeugs 12 und Pfeil 15 die Öffnungsbewegung des Greifwerkzeugs 12. In dem stiftförmigen Gehäuse 11 ist in Richtung der Längsachse eine als Druckstift 17 ausgebildete Druckvorrichtung angeordnet, die auf eine Spreizvorrichtung 16 zum Spreizen des Greifwerkzeugs und eine Drehvorrichtung 18 zum Drehen des Greifwerkzeugs wirkt. Eine im Innern des Gehäuses 11 angeordnetes Federelement 19 stellt die notwendige Kraft bereit, um den die im Folgenden beschriebene Wirkungsweise des Zeckenfängers 10 bereitzustellen.

Der Zeckenfänger 10 funktioniert ähnlich einem Druckbleistift. Bei diesem spreizen sich auf Daumendruck Greiflamellen an der Spitze des Stiftes, um die Bleistiftmine frei zu geben.

In dem ersten Ausführungsbeispiel nach Fig. 1 öffnet die Spreizvorrichtung 16 bei einem Daumendruck auf den Druckstift 17 in Richtung der Längsachse in das innere des Gehäuses hinein, wie dies durch den Pfeil 20 symbolisiert ist und einer ersten Betätigung entspricht, ein an der Spitze geschlitztes Greifwerkzeug 12, was beispielsweise durch ein Silikonbällchen gebildet sein kann. Dieses Greifwerkzeug 12 wird über der Zecke positioniert und so auf die Haut aufgesetzt, dass sich die Zecke in der Öffnung des Greifwerkzeugs 12 befindet. Mit Rücknahme des Daumendruckes auf den Druckstift 17, d.h. Bewegung des Druckstiftes entgegengesetzt zur Pfeilrichtung 20 entsprechend einer zweiten Betätigung, fährt die Spreizvorrichtung 16 zurück und das Greifwerkzeug 12 umschließt die Zecke in dem Hohlraum 21. Weiteres Nachlassen des Daumendruckes auf den Druckstift bewirkt eine Drehung des Greifwerkzeugs 12 durch die Drehvorrichtung 18, wodurch die Zecke aus der Haut gelöst und vom Greifwerkzeug 12 aufgenommen wird. Dieses Greifwerkzeug 12 ist lösbar an der Vorrichtung 10 angeordnet und kann abgenommen werden, um es bei Bedarf mitsamt der Zecke an ein Labor zur Untersuchung einzuschicken. Aus hygienischen Gründen wird die Einmalverwendung der Greifwerkzeuge 12 bevorzugt .

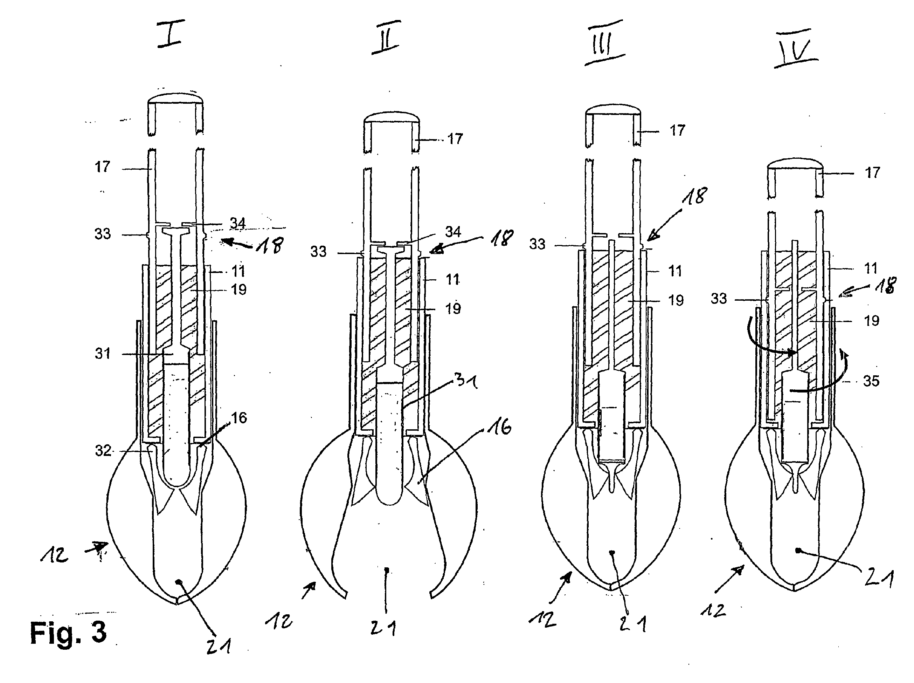

Fig. 3 zeigt eine zweite Ausführungsform des erfindungsgemäßen Zeckenfängers 30 in schematischer Darstellung, wobei Fig. 3 den Zeckenfänger in vier Zuständen , II, III, und IV darstellt.

Die zweite Ausführungsform des Zeckenfängers 30 arbeitet ebenfalls wie die erste Ausführungsform vergleichbar einem Druckbleistift, wobei der Zeckenfänger 30 ein Gehäuse 11, ein spreizbares und drehbares Greifwerkzeug 12 sowie einen Druckstift 17 aufweist. Die Dreh- und Spreizrichtungen des Greifwerkzeugs 12 sowie das Greifwerkzeug 12 der zweiten Ausführungsform entsprechen der ersten Ausführungsform, so dass sich eine erneute Beschreibung erübrigt. Ferner weist die zweite Ausführungsform eine Drehvorrichtung 18 sowie eine Feder 19 zur Energiespeicherung und Bereitstellung der notwendigen Kräfte auf.

Im Zustand 1 des Zeckenfängers 30 der Fig. 1, welcher der Ruheposition entspricht, ist das Greifwerkzeug 12 geschlossen und umschließt vollständig einen inneren Hohlraum 21, dessen Größe für eine sichere Aufnahme des Parasiten ausreichend ist. Der Druckbolzen 31 der Spreizvorrichtung 16 ist ebenfalls in der Ruheposition und ist in einer Ausnehmung zwischen geeignet geformten Spreizbacken 32 zur Spreizung des Greifwerkzeugs 12 angeordnet.

Der Zustand II der Fig. 1 zeigt den Zeckenfänger 30 während oder nach einer ersten Betätigung des Druckstiftes, d.h. der Druckstift 17 wird nach innen entlang der Längsachse des Zeckenfängers 30 gedrückt. Durch die Bewegung des Druckstiftes 17 in der Fig. 3 nach unten wird der auf Grund seiner Nutführung quer stehende Druckbolzen 31 der Spreizvorrichtung 16 ebenfalls durch einen Mitnehmer 34 nach unten verschoben, was zu einer Spreizung, d.h. Öffnung, des Greifwerkzeugs 12 durch die Wirkung des Druckbolzens 31 auf die Spreizbacken 32 führt.

Im Zustand III des Zeckenfängers 30 wurde der Druck auf den Druckstift in die gleiche Richtung wie im Zustand II fortgesetzt. Dies kann als Endposition einer ersten Betätigung oder als Beginn einer zweiten Betätigung betrachtet werden, da bei der zweiten Ausführungsform des Zeckenfängers 30 die erste und zweite Betätigung nahtlos ineinander übergehen können. Bedingt durch die Nutführung wird der Druckbolzen 31 jetzt um 90° um seine Längsachse gedreht, wodurch durch die Ausgestaltung des Druckbolzens, d.h. durch die dargestellte Abflachung, sich das Greifwerkzeug 12 durch eigene Federkraft schließt. Da auch das obere Ende des Druckbolzens 31 entsprechend abgeflacht gestaltet ist, liegt dieses jetzt in der Öffnung des Mitnehmers 34 des Druckstifts 17 und kann somit nicht mehr weiter gedrückt werden, d.h. der Druckbolzen 31 hat seine Endposition erreicht.

Der Zustand IV des Zeckenfängers 30 wird durch weitere Betätigung des Druckstiftes 17 in die gleiche Richtung entlang der Längsachse der Vorrichtung 30 erreicht. Die weitere Betätigung in die gleiche Richtung wie die erste Betätigung bewirkt eine Drehung oder Rotation des geschlossenen Greifwerkzeugs 17, symbolisch dargestellt durch den Pfeil 35. Die Drehung des Greifwerkzeugs 17 wird bewirkt durch eine Drehvorrichtung 18, die gebildet wird durch zwei auf der Außenseite des Druckstifts 17 angeordnete Noppen 33, die jetzt in das Innengewinde des Gehäuses 11 greifen. Bei weiterem Druck auf den Druckstift 17 wird somit das Gehäuse 11, auf dem das Greifwerkzeug 12 befestigt ist, gedreht.

Vorteilhaft bei der zweiten Ausführungsform ist, dass der Zeckenfänger das Greifwerkzeug öffnet, schließt und dreht durch Betätigen oder Einfahren des Druckstiftes in eine Richtung. Der Bewegungsablauf ist abgeschlossen, wenn der Druckstift vollständig eingedrückt ist. Im Gegensatz zur ersten Ausführungsform 1 wird durch die unidirektionale Betätigung des Druckstiftes ein Lastwechsel vermieden. Dadurch lässt sich ein "Verzittern", insbesondere bei der Entfernung kleinster Zecken, verhindern.

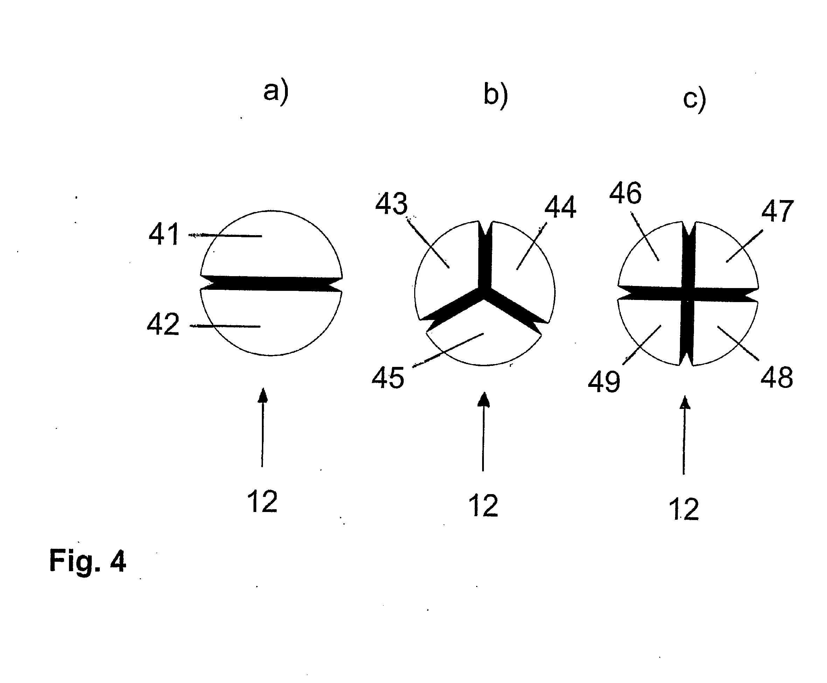

Fig. 4 zeigt Varianten des Greifwerkzeugs 12 in schematischer Ansicht von unten. In Teil a) der Fig. 4 ist ein Greifwerkzeug bestehend aus zwei Segmenten 41, 42, d.h. sog. 180° Sektoren, in Teil b) ein Greifwerkzeug bestehend aus drei Segmenten 43, 44, 45, d.h. sog. 120° Sektoren, und in Teil c) ein Greifwerkzeug bestehend aus vier Segmenten 46, 47, 48, 49, d.h. sog. 90° Sektoren, dargestellt. Zur besseren bildlichen Darstellung sind die verschiedenen Greifwerkzeuge leicht geöffnet dargestellt, um einen Endruck vom Verlauf der Schnittkanten zu erreichen. Im geschlossenen Zustand liegen die Kanten der Segmente aneinander an.

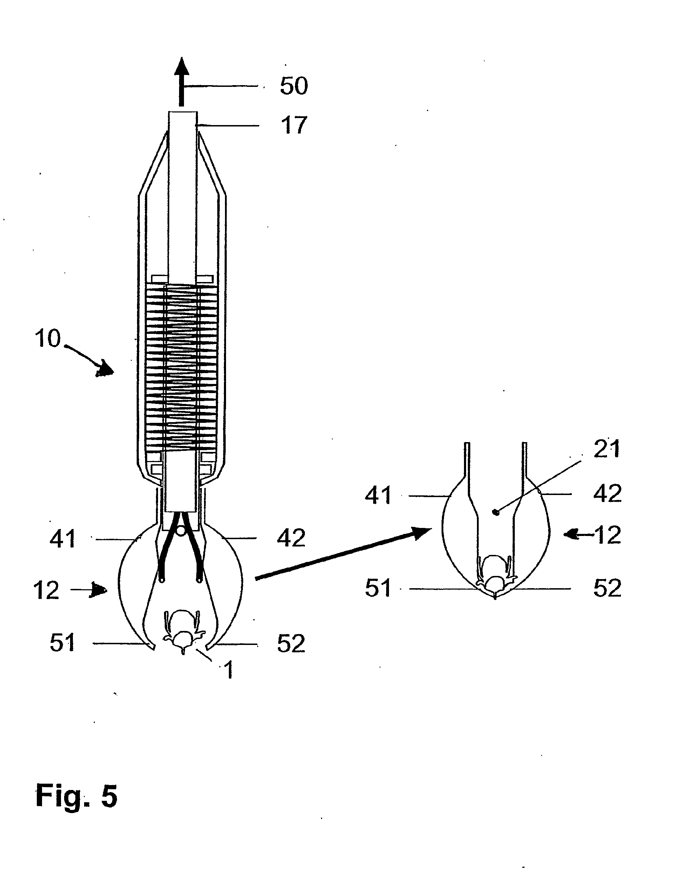

Fig. 5 zeigt schematisch die Anwendung der ersten Ausführungsform des erfindungsgemäßen Zeckenfängers 10 mit einem Greifwerkzeug 12 bestehend aus zwei Segmenten 41, 42 in schematischer Querschnittsansicht entlang der Längsachse der Vorrichtung 10, d.h. des Zeckenfängers 10. Der Zeckenfänger 10 wird in geöffnetem Zustand des Greifwerkzeugs 12 über der Zecke 1 auf die Haut des Wirts platziert. Anschließend wird durch Loslassen des Druckstifts 17 in Pfeilrichtung 50 ein Schließen der Segmente 41 und 42 sowie eine Rotation des Greifwerkzeugs 12 bewirkt, wodurch die Zecke aus der Haut des Wirts gelöst und abgehoben wird. Die Zecke 1 befindet sich dann vollständig im geschlossenen Hohlraum 21 des Greifwerkzeugs 12, wie dies im rechten Teil der Fig. 5 dargestellt ist. Im Querschnitt der Segmente 41 und 42 des Greifwerkzeugs ist ferner zu erkennen, dass sich die Dicke der Segment zur Greifwerkzeugspitze hin verringert, d.h. ein Segment 41, 42 verjüngt sich dickenmäßig zur Segmentspitze hin. Die durch die Verjüngung gebildeten Segmentbacken 51, 52 sind ferner nach innen gerichtet, um einerseits die Zecke so nah wie möglich am Wirt zu fassen und andererseits im geschlossenen Zustand den Hohlraum 21 zu bilden. Nicht dargestellt ist, dass die Verjüngung der Wandstärke der Segmente zur Greifwerkzeugspitze hin auch bei den Greifwerkzeugen mit mehr als zwei Segmenten, wie sie in Fig. 4 schematisch dargestellt sind, vorhanden ist. Ferner wird für das Greifwerkzeug ein elastisches bzw. flexibles Material verwendet, um eine Quetschung der Zecke zu vermeiden. Weiterhin kann das Greifwerkzeug mit der Drehvorrichtung lösbar, beispielsweise mittels eines Bajonetverschluß, verbunden sein (nicht dargestellt), so dass das Greifwerkzeug nach einmaliger Verwendung entfernt werden kann. Ferner kann dass das Greifwerkzeug einen Feuchtigkeitsspender (nicht dargestellt) aufweisen, so dass die Zecke im Greifwerkzeug einige Zeit überleben kann, falls das Greifwerkzeug mit der lebenden Zecke zu einer Untersuchung geschickt wird. Der Feuchtigkeitsspender kann im einfachsten Fall durch das Einbringen eines Tropfen Wassers in das Greifwerkzeug gebildet werden.

Ferner kann mit Beendigung der Zeckenentfernung, d.h. wenn sich die lebende Zecke im Hohlraum befindet, das Greifwerkzeug 12 von der Drehvorrichtung des Zeckenfängers mittels einer nicht dargestellten Abwurfvorrichtung gelöst, d.h. abgeworfen, werden. Das abgeworfene Greifwerkzeug mit der darin befindlichen Zecke kann dann zur Untersuchung der Zecke hinsichtlich ihrer Infektion versendet werden.

### BEZUGSZEICHENLISTE

- 1: Zecke
- 2: Pinzette
- 3: Wirtshaut
- 4: Pinzettenarm
- 5: Pinzettenarm

- 10: Zeckenfänger
- 11: Gehäuse
- 12: Greifwerkzeug
- 13: Drehrichtung
- 14: Spreizrichtung
- 15: Öffnungsrichtung
- 16: Spreizvorrichtung
- 17: Druckstift
- 18: Drehvorrichtung
- 19: Feder
- 20: Richtung des Eindrücken des Druckstifts
- 21: Hohlraum

- 30: Zeckenfänger
- 31: Druckbolzen
- 32: Spreizbacken
- 33: Noppen
- 34: Mitnehmer
- 35: Drehrichtung Greifwerkzeug

- 41: 180°-Segment
- 42: 180°-Segment
- 43: 120°-Segment
- 44: 120°-Segment
- 45: 120°-Segment
- 46: 90°-Segment
- 47: 90°-Segment
- 48: 90°-Segment
- 49: 90°-Segment

- 50: Richtung zum Ausfahren des Druckstiftes
- 51: Segmentbacke
- 52: Segmentbacke

## Patentansprüche

1. Vorrichtung (10, 30), insbesondere zum Entfernen von Parasiten oder Zecken (1) aus der Haut von Tieren und Menschen, mit einem Gehäuse (11), einem spreizbaren Greifwerkzeug (12), einer Spreizvorrichtung(16) zum Spreizen des Greifwerkzeugs (12) und einer Drehvorrichtung (18) zur Drehung des Greifwerkzeugs (12) um die Längsachse der Vorrichtung,
**dadurch gekennzeichnet, dass**
das Greifwerkzeug (12) in seinem ungespreizten Zustand einen im wesentlichen geschlossenen Hohlraum (21) zur Aufnahme des Parasiten oder der Zecke (1) umschließt, und dass
die Vorrichtung (10, 30) weiterhin eine in axialer Richtung der Vorrichtung wirkende Druckvorrichtung (17) zur Betätigung der Spreizvorrichtung (16) und der Drehvorrichtung (18) des Greifwerkzeugs (12) aufweist.

2. Vorrichtung nach Anspruch 1, **dadurch gekennzeichnet, dass** eine erste Betätigung der Druckvorrichtung (17) eine Spreizung der Greifwerkzeugs (12) durch die Spreizvorrichtung (16) bewirkt.

3. Vorrichtung nach Anspruch 2, **dadurch gekennzeichnet, dass** eine zweite Betätigung der Druckvorrichtung (17) eine Drehung des Greifwerkzeugs (12) bewirkt.

4. Vorrichtung nach einem der Ansprüche 2 oder 3, **dadurch gekennzeichnet, dass** eine Schließung des Greifwerkzeugs (12) durch die erste oder zweite Betätigung der Druckvorrichtung (17) bewirkt wird.

5. Vorrichtung nach einem der Ansprüche 1 bis 4, **dadurch gekennzeichnet, dass** die erste Betätigung der Druckvorrichtung (17) in axialer Richtung in die Vorrichtung (10, 30) hinein erfolgt und die sich an die erste Betätigung anschließende zweite Betätigung in axialer Richtung aus der Vorrichtung (10, 30) heraus erfolgt.

6. Vorrichtung nach einem der Ansprüche 1 bis 4, **dadurch gekennzeichnet, dass** die erste Betätigung der Druckvorrichtung (17) in axialer Richtung in die Vorrichtung (10, 30) hinein und die sich an die erste Betätigung anschließende zweite Betätigung weiter in axialer Richtung in die Vorrichtung (10, 30) hinein erfolgt.

7. Vorrichtung nach einem der vorangegangenen Ansprüche, **dadurch gekennzeichnet, dass** das Greifwerkzeug (12) aus zwei oder mehreren Segmenten (41, 42; 43, 44, 45; 46, 47, 48, 49) gebildet ist.

8. Vorrichtung nach Anspruch 7, **dadurch gekennzeichnet, dass** das Greifwerkzeug (12) aus zwei, drei oder vier Segmenten (41, 42; 43, 44, 45; 46, 47, 48, 49) gebildet ist.

9. Vorrichtung nach Anspruch 7 oder 8, **dadurch gekennzeichnet, dass** die Segmente (41, 42; 43, 44, 45; 46, 47, 48, 49) des Greifwerkzeugs (12) elastisch sind.

10. Vorrichtung nach einem der Ansprüche 7 bis 9, **dadurch gekennzeichnet, dass** die Segmente (41, 42; 43, 44, 45; 46, 47, 48, 49) des Greifwerkzeugs (12) zur Spitze des Greifwerkzeugs (12) hin sich verjüngende und nach innen gerichtete Greifbacken 51, 52) bilden.

11. Vorrichtung nach einem der Ansprüche 7 bis 10, **dadurch gekennzeichnet, dass** die Segmente (41, 42; 43, 44, 45; 46, 47, 48, 49) des Greifwerkzeugs (12) eine Federkraft aufweisen, gegen welche die Spreizung der Segmente (41, 42; 43, 44, 45; 46, 47, 48, 49) erfolgt.

12. Vorrichtung nach einem der vorangegangenen Ansprüche, **dadurch gekennzeichnet, dass** das Greifwerkzeug (12) mit einem Klebstoff beschichtet ist.

13. Vorrichtung nach einem der vorangegangenen Ansprüche, **dadurch gekennzeichnet, dass** das Greifwerkzeug (12) Widerhaken aufweist.

14. Vorrichtung nach einem der vorangegangenen Ansprüche, **dadurch gekennzeichnet, dass** die Vorrichtung (10, 30) eine mit dem Greifwerkzeug (12) verbundenen Saugeinrichtung aufweist.

15. Vorrichtung nach einem der vorangegangenen Ansprüche, **dadurch gekennzeichnet, dass** die Vorrichtung (10, 30) eine Einrichtung zum Lähmen oder Abtöten der Parasiten (1) aufweist.

16. Vorrichtung nach einem der vorangegangenen Ansprüche, **dadurch gekennzeichnet, dass** das Greifwerkzeug (12) austauschbar ist.

17. Vorrichtung nach Anspruch 16, **dadurch gekennzeichnet, dass** das Greifwerkzeug (12) in verschiedenen Größen und Materialstärken zum Austausch zur Verfügung steht.

18. Vorrichtung nach einem der Ansprüche 16 oder 17, **dadurch gekennzeichnet, dass** das Greifwerkzeug einen Feuchtigkeitsspender aufweist.

19. Vorrichtung nach einem der Ansprüche 16 bis 18, **dadurch gekennzeichnet, dass** die Vorrichtung eine Abwurfeinrichtung zum Abwerfen des Greifwerkzeugs aufweist

20. Vorrichtung nach Anspruch 19, **dadurch gekennzeichnet, dass** die Abwurfeinrichtung das Greifwerkzeug mit Beendigung des Vorgangs der Entfernung eines Parasiten von der Vorrichtung löst.

## Claims

1. Device (10, 30), in particular for the removal of parasites or ticks (1) from the skin of animals and humans, with a housing (11), a spreadable grippertool (12), a spreader device (16) for spreading the gripper tool (12) and a rotation device (18) for the rotation of the gripper tool (12) around the longitudinal axis of the device,
**characterized in that**
the gripper tool (12) in its un-spread position encloses an essentially closed cavity (21) that is designed to contain the parasite or the tick (1) whereby the device (10, 30) also has a presser device (17) that acts in the axial direction of the device to actuate the spreader device (16) and the rotation device (18) of the gripper tool (12).

2. Device as claimed in Claim 1, **characterized in that** a first actuation of the presser device (17) effects a spreading of the grippertool (12) by the spreading device (16).

3. Device as claimed in Claim 2, **characterized in that** a second actuation of the presser device (17) effects a rotation of the grippertool (12).

4. Device as claimed in one of the Claims 2 or 3, **characterized in that** a closing of the gripper tool (12) is effected by the first or second actuation of the presser device (17).

5. Device as claimed in one of the Claims 1 to 4, **characterized in that** the first actuation of the presser device (17) takes place in the axial direction into the device (10, 30) and the second actuation that follows the first actuation takes place in the axial direction out of the device (10, 30).

6. Device as claimed in one of the Claims 2, to 5, **characterized in that** the first actuation of the presser device (17) takes place in the axial direction into the device (10, 30) and the second actuation that follows the first actuation continues in the axial direction into the device (10, 30).

7. Device as claimed in one of the preceding claims, **characterized in that** the gripper tool (12) is formed by two or more segments, (41, 42; 43,44, 45; 46,47, 48, 49).

8. Device as claimed in Claim 7, **characterized in that** the gripper tool (12) is formed by two, three or four segments (41, 42; 43, 44, 45; 46, 47, 48, 49).

9. Device as claimed in Claim 7 or 8, **characterized in that** the segments (41,42; 43, 44,45; 46, 47, 48,49) of the gripper tool (12) are elastic.

10. Device as claimed in one of the Claims 7 to 9, **characterized in that** the segments (41, 42; 43, 44, 45; 46, 47, 48, 49) of the gripper tool (12) are tapered toward the tip of the gripper tool (12) and form inward-facing gripper jaws (51, 52).

11. Device as claimed in one of the Claims 7 to 10, **characterized in that** the segments (41, 42;, 43, 44, 45; 46, 47, 48, 49) of the gripper tool (12) have a spring force, against which the spreading of the segments (41, 42; 43, 44, 45; 46, 47, 48, 49) takes place.

12. Device as claimed in one of the preceding claims, **characterized in that** the gripper tool (12) is coated with an adhesive.

13. Device as claimed in one of the preceding claims, **characterized in that** the gripper tool (12) is provided with barbs.

14. Device as claimed in one of the preceding claims, **characterized in that** the device (10, 30) has a suction device that is connected with the gripper tool (12).

15. Device as claimed in one of the preceding claims, **characterized in that** the device (10, 30) has an apparatus to paralyze or kill the parasite (1).

16. Device as claimed in one of the preceding claims, **characterized in that** the grippertool (12) is interchangeable and replaceable.

17. Device as claimed in Claim 16, **characterized in that** the gripper tool (12) is available in interchangeable different sizes and material thicknesses.

18. Device as claimed in one of the Claims 16 or 17, **characterized in that** the gripper tool has a moisture dispenser.

19. Device as claimed in one of the Claims 16 to 18, **characterized in that** the device has an ejector device for the ejection of the gripper tool.

20. Device as claimed in Claim 19, **characterized in that** the ejector device detaches the gripper tool from the device when the process of removing the parasite is completed.

## Revendications

1. Dispositif (10, 30), permettant notamment de retirer des parasites ou des tiques (1) hors de la peau des animaux et des êtres humains, avec un corps (11), un instrument de saisie expansible (12), un dispositif d'expansion (16) pour expanser l'instrument de saisie (12), et un dispositif rotatif (18) pour faire tourner l'instrument de saisie (12) autour de l'axe longitudinal du dispositif, **caractérisé en ce que** l'instrument de saisie (12) comprend, à l'état non expansé, un espace creux (21) essentiellement fermé destiné à recevoir le parasite ou la tique (1), et **en ce que** le dispositif (10, 30) comprend en outre un dispositif de pression (17) agissant dans la direction axiale du dispositif pour actionner le dispositif d'expansion (16) et le dispositif rotatif (18) de l'instrument de saisie (12).

2. Dispositif selon la revendication 1, **caractérisé en ce qu'**un premier actionnement du dispositif de pression (17) provoque une expansion de l'instrument de saisie (12) par le dispositif d'expansion (16).

3. Dispositif selon la revendication 2, **caractérisé en ce qu'**un deuxième actionnement du dispositif de pression (17) provoque une rotation de l'instrument de saisie (12).

4. Dispositif selon l'une des revendications 2 ou 3, **caractérisé en ce qu'**une fermeture de l'instrument de saisie (12) est provoquée par le premier ou le deuxième actionnement du dispositif de pression (17).

5. Dispositif selon l'une quelconque des revendications 1 à 4, **caractérisé en ce que** le premier actionnement du dispositif de pression (17) est effectué en direction axiale vers l'intérieur du dispositif (10, 30) et le deuxième actionnement faisant suite au premier actionnement est effectué en direction axiale vers l'extérieur du dispositif (10, 30).

6. Dispositif selon l'une quelconque des revendications 1 à 4, **caractérisé en ce que** le premier actionnement du dispositif de pression (17) est effectué en direction axiale vers l'intérieur du dispositif (10, 30) et le deuxième actionnement faisant suite au premier actionnement est effectué en direction axiale plus profondément vers l'intérieur du dispositif (10, 30).

7. Dispositif selon l'une quelconque des revendications précédentes, **caractérisé en ce que** l'instrument de saisie (12) est formé de deux ou de plusieurs segments (41, 42; 43, 44, 45; 46, 47, 48, 49).

8. Dispositif selon la revendication 7, **caractérisé en ce que** l'instrument de saisie (12) est formé de deux, trois ou quatre segments (41, 42; 43, 44, 45; 46, 47, 48, 49).

9. Dispositif selon la revendication 7 ou 8, **caractérisé en ce que** les segments (41, 42; 43, 44, 45; 46, 47, 48, 49) de l'instrument de saisie (12) sont élastiques.

10. Dispositif selon l'une quelconque des revendications 7 à 9, **caractérisé en ce que** les segments (41, 42; 43, 44, 45; 46, 47, 48, 49) de l'instrument de saisie (12) forment des mâchoires de saisie (51, 52) orientées vers l'intérieur et s'amenuisant en direction de la pointe de l'instrument de saisie (12).

11. Dispositif selon l'une quelconque des revendications 7 à 10, **caractérisé en ce que** les segments (41, 42; 43, 44, 45; 46, 47, 48, 49) de l'instrument de saisie (12) présentent une force de ressort, contre laquelle on effectue l'expansion des segments (41, 42; 43, 44, 45; 46, 47, 48, 49).

12. Dispositif selon l'une quelconque des revendications précédentes, **caractérisé en ce que** l'instrument de saisie (12) est revêtu d'une colle.

13. Dispositif selon l'une quelconque des revendications précédentes, **caractérisé en ce que** l'instrument de saisie (12) comporte des barbes.

14. Dispositif selon l'une quelconque des revendications précédentes, **caractérisé en ce que** le dispositif (10, 30) comprend un dispositif d'aspiration relié à l'instrument de saisie (12).

15. Dispositif selon l'une quelconque des revendications précédentes, **caractérisé en ce que** le dispositif (10, 30) comprend un système permettant d'immobiliser ou de tuer les parasites (1).

16. Dispositif selon l'une quelconque des revendications précédentes, **caractérisé en ce que** l'instrument de saisie (12) est remplaçable.

17. Dispositif selon la revendication 16, **caractérisé en ce que** l'instrument de saisie (12) est disponible en différentes tailles et épaisseurs en vue de son remplacement.

18. Dispositif selon l'une des revendications 16 ou 17, **caractérisé en ce que** l'instrument de saisie comprend un distributeur d'humidité.

19. Dispositif selon l'une quelconque des revendications 16 à 18, **caractérisé en ce que** le dispositif comprend un système d'éjection permettant d'éjecter l'instrument de saisie.

20. Dispositif selon la revendication 19, **caractérisé en ce que** le système d'éjection détache l'instrument de saisie du dispositif à la fin de l'opération d'enlèvement d'un parasite.
